(19) **Europäisches Patentamt**

**European Patent Office**

**Office européen des brevets**

(11) **EP 1 072 222 B1**

(12) **EUROPÄISCHE PATENTSCHRIFT**

(45) Veröffentlichungstag und Bekanntmachung des Hinweises auf die Patenterteilung:
**02.06.2004 Patentblatt 2004/23**

(51) Int Cl.$^7$: **A61B 5/00**, G01N 33/487

(21) Anmeldenummer: **00115207.3**

(22) Anmeldetag: **13.07.2000**

(54) **Anordnung zur Konzentrationsbestimmung von Glucose in einer Körperflüssigkeit**

Apparatus for determination of glucose concentration in body fluid

Appareil pour déterminer la concentration de glucose dans un fluide anatomique

(84) Benannte Vertragsstaaten:
**AT BE CH CY DE DK ES FI FR GB GR IE IT LI LU MC NL PT SE**

(30) Priorität: **28.07.1999 DE 19935165**

(43) Veröffentlichungstag der Anmeldung:
**31.01.2001 Patentblatt 2001/05**

(73) Patentinhaber: **Roche Diagnostics GmbH**
**68305 Mannheim (DE)**

(72) Erfinder:
• **Gessler, Ralf**
**88255 Baienfurt (DE)**
• **Hoss, Udo**
**Valencia, CA 91355 (US)**
• **Zieten, Hans-Ulrich**
**89186 Illerrieden (DE)**
• **Pfleiderer, Hans-Jörg**
**89075 Ulm (DE)**
• **Fussgaenger, Rolf**
**89134 Blaustein-Weidach (DE)**

(74) Vertreter: **Pfiz, Thomas, Dr. et al**
**Patentanwälte Wolf & Lutz**
**Hauptmannsreute 93**
**70193 Stuttgart (DE)**

(56) Entgegenhaltungen:
**WO-A-97/42868          DE-A- 4 401 400**

**Beschreibung**

Die Erfindung betrifft eine Anordnung zur Konzentrationsbestimmung von Glucose in einer Körperflüssigkeit, insbesondere Gewebeflüssigkeit, gemäß dem Oberbegriff des Patentanspruchs 1 bzw. 19.

Eine Anordnung dieser Art ist aus der WO 97/42868 bekannt. Dort werden intermittierende Förderschübe vorgeschlagen, um einerseits eine fortlaufende Signalkalibrierung zu ermöglichen und andererseits den Meßvorgang zu beschleunigen. Dabei gleicht sich während Ruhephasen zwischen den Förderschüben das momentan in der Mikrodialysesonde befindliche Perfusatvolumen aufgrund des Dialysevorgangs an die Konzentration der Gewebeglucose an, während angrenzende Volumenbereiche in der nachfolgend mit hoher Fließgeschwindigkeit weitertransportierten Flüssigkeitssäule weitgehend unverändert bleiben. Entsprechend dem Konzentrationsgradient wird an der Meßzelle eine Signalspitze während eines Förderschubs beobachtet, aus welcher der Glucosegehalt des Dialysats und damit auch der Körperflüssigkeit bestimmbar ist. Zur Kalibrierung wird glucosehaltige Perfusionsflüssigkeit eingesetzt, deren vorgegebene Glucosekonzentration den Grundlinienwert der Signalspitze definiert. Dabei wird neben einem vollständigen Konzentrationsausgleich während der Dialysephasen ein lineares Sensorverhalten vorausgesetzt, und es wird angenommen, daß das Konzentrationsprofil im dem aus der Sonde abtransportierten Volumen bis zur Meßzelle nicht zerfällt. Besonders die letzte Annahme trifft jedoch häufig nicht zu, weil insbesondere bei laminarer Strömung eine Durchmischung stattfindet. Hinzu kommt, daß durch Diffusionsaustausch das Glucosegleichgewicht in dem die Sonde umgebenden Gewebe gestört wird.

Ausgehend hiervon liegt der Erfindung die Aufgabe zugrunde, die vorgenannten Nachteile und insbesondere Konzentrationsstörungen in der Körperflüssigkeit zu vermeiden und eine exakte Glucosebestimmung bei reduzierter Dialysedauer zu ermöglichen.

Zur Lösung dieser Aufgabe werden die in den Patentansprüchen 1 und 19 angegebenen Merkmalskombinationen vorgeschlagen. Vorteilhafte Ausgestaltungen und Weiterbildungen der Erfindung ergeben sich aus den abhängigen Ansprüchen.

Der Erfindung liegt der Gedanke zugrunde, den Glucosegehalt des Perfusats selbsteinstellend bzw. adaptiv an die Glucosekonzentration der Körperflüssigkeit anzupassen. Dementsprechend wird zur Lösung der vorstehend genannten Aufgabe eine Regeleinrichtung zur Angleichung des Ausgangsgehalts der Glucose im Perfusat an den Glucosegehalt der Körperflüssigkeit nach Maßgabe einer aus den Meßsignalen der Meßzelle abgeleiteten Führungsgröße vorgeschlagen. Damit werden Glucosegradienten ausgeglichen und entsprechend die erforderliche Zeitdauer für einen vollständigen Dialyseausgleich verringert. Weiter werden auch bei hoher Durchströmung der Mikrodialysesonde und Glucoseschwankungen der Körperflüssigkeit auf Glucosegradienten beruhende Störeffekte vermieden.

In bevorzugter Ausgestaltung ist eine Auswerteinheit vorgesehen, mittels welcher der Glucosegehalt der Körperflüssigkeit entsprechend dem momentanen Ausgangsgehalt der Glucose im Perfusat bei verschwindender Regelabweichung bestimmt wird. Damit ist es möglich, eine quantitative Konzentrationsbestimmung über den momentanen Ist-Wert der Regelgröße mittelbar vorzunehmen, während die fortlaufend abgegriffenen Meßsignale der Meßzelle lediglich als Regiereingangsgrößen verwertet werden. Alternativ oder ergänzend ist es grundsätzlich möglich, daß aus den Meßsignalen unmittelbar der Glucosegehalt der Körperflüssigkeit abgeleitet wird.

Vorteilhafterweise wird der Ausgangsgehalt der Glucose im Perfusat aus der Störgröße des Stellglieds der Regeleinrichtung ermittelt. Mit dieser Maßnahme kann der Ausgangsgehalt beispielsweise durch Vergleich mit normierten Tabellenwerten exakt ermittelt werden, ohne daß zusätzliche Glucosesensoren erforderlich wären. Prinzipiell ist es jedoch auch möglich, daß der Glucosegehalt des Perfusats vor der Einleitung in die Mikrodialysesonde gemessen wird.

Die Perfusionseinrichtung umfaßt einen Perfusatvorrat sowie eine Fördereinheit zur Förderung von Perfusat. Die Fördereinheit arbeitet vorzugsweise intervallweise, d.h. mit unterschiedlichem Förderstrom in aufeinanderfolgenden Zeitintervatten. Um den Glucoseausgangsgehalt variieren zu können, ist es von Vorteil, wenn der Perfusatvorrat mindestens zwei gesonderte Reservoirs zur Aufnahme von Perfusionsflüssigkeiten mit voneinander verschiedener Glucosekonzentration aufweist. Vorteilhafterweise weist der Perfusatvorrat ein glucosefreie Perfusionsflüssigkeit enthaltendes erstes Reservoir und ein glucosehaltige Perfusionsflüssigkeit enthaltendes zweites Reservoir auf. Dabei sollte in letzterem der Glucosegehalt oberhalb der physiologischen Grenzwerte liegen. Eine baulich einfache Stelleinrichtung zur Einstellung des Ausgangsgehalts der Glucose im Perfusat sieht einen vorzugsweise durch ein Mischventil oder getaktet schaltbares Wegeventil gebildeten Strömungsmischer als Stellglied vor. Dabei ist es günstig, wenn der Strömungsmischer zuströmseitig mit mindestens zwei Reservoirs zur Zuleitung von Perfusionsflüssigkeiten mit voneinander verschiedenem Glucosegehalt verbunden ist und abströmseitig in eine zu der Mikrodialysesonde führende Perfusatleitung mündet.

Zur variablen Verarbeitung des Signalflusses weist die Regeleinrichtung vorteilhafterweise einen digital arbeitenden, vorzugsweise durch einen Mikrocontroller gebildeten Regler auf.

Gemäß einer besonders bevorzugten Ausgestaltung der Erfindung ist es vorgesehen, daß das Perfusat in alternierend aufeinanderfolgenden Transport- und Dialyse-Intervallen mit unterschiedlicher Fließgeschwindigkeit durch die Mikrodialysesonde hindurchgeleitet wird, wobei die Fließgeschwindigkeit während der Transportintervalle höher ist als während der Dialyseintervalle. Dadurch kann die Messung insgesamt verkürzt und die Auswertung weiter vereinfacht werden, da ein vorhandener Konzentrationsgradient auch bei nur teilweisem Dialyseausgleich über das Meßsignal qualitativ erkennbar ist. Dabei sollte die Fließgeschwindigkeit während der Transportintervalle so weit erhöht werden, daß der Ausgangsgehalt der Glucose im Perfusat beim Durchgang durch die Mikrodialysesonde im wesentlichen erhalten bleibt. Hingegen wird während der Dialyseintervalle die Förderung unterbrochen oder zumindest die Fließgeschwindigkeit so verringert, daß die Glucosekonzentration im Dialysat an den Glucosegehalt der Körperflüssigkeit angenähert wird.

Eine besonders einfache Regelung sieht vor, daß die den Sollwert festlegende Führungsgröße durch Integration oder Differentiation des zeitlichen Verlaufs der Meßsignale oder durch eine qualitative Erkennung von Signalspitzen im zeitlichen Verlauf der Meßsignale ermittelt wird. Alternativ kann die Führungsgröße durch Vergleich des aktuellen Signalverlaufs der Meßsignale mit in einem Speichermittel hinterlegten kalibrierten Signalmustern ermittelt werden. Eine weitere Möglichkeit besteht darin, daß die Führungsgröße aus dem Spitzenwert des Signalverlaufs der Meßsignale während eines jeden Transportintervalls ermittelt wird. Um das Reglereingangssignal quantitativ zu definieren, kann die Führungsgröße - entsprechend dem Glucosegehalt c der Körperflüssigkeit - gemäß der Beziehung

$$c = \left[ \frac{S_g}{S_g \cdot (1-b) + b \cdot S_0} - 1 \right] \cdot a \cdot c_0 + c_0$$

bestimmt werden, wobei $S_g$ den Spitzenwert und $S_0$ den Grundlinienwert der Meßsignale während eines Transportintervalls, $c_0$ den momentanen Ausgangsgehalt der Glucose im Perfusat und a, b empirisch bestimmte Korrekturfaktoren zur Kompensation von Diffusions- und Mischungseffekten sowie verbleibenden Angleichungs- bzw. Recovery-Effekten zwischen Körper- und Perfusionsflüssigkeit während des Transportintervalls bezeichnen.

Eine besonders einfache Regierfunktion sieht vor, daß der Ausgangsgehalt der Glucose im Perfusat durch einen Zweipunktregelvorgang unstetig geregelt wird, wobei bei einer Regelabweichung der Ausgangsgehalt der Glucose im Perfusat um einen vorgegebenen Stellwert verändert wird.

Für eine variable Einstellung ist es von Vorteil, wenn der Ausgangsgehalt der Glucose im Perfusat durch Strömungsmischen von zwei in getrennten Reservoirs mit voneinander verschiedener Glucosekonzentration bereitgehaltenen Perfusionsflüssigkeiten beeinflußt wird.

Im folgenden wird die Erfindung anhand eines in der Zeichnung in schematischer Weise dargestellten Ausführungsbeispiels näher erläutert. Es zeigen

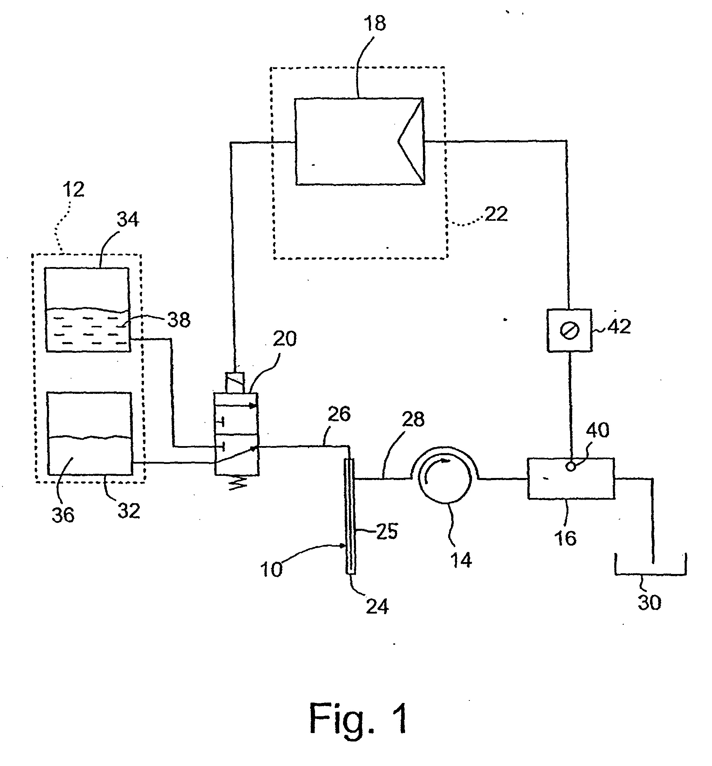

Fig. 1 ein Blockschaltbild einer Mikrodialyseanordnung zur Konzentrationsbestimmung von Glucose in Gewebeflüssigkeit; und

Fig. 2 ein Zeitdiagramm des Perfusatstroms, des Glucose-Meßsignals im Dialysat und der adaptiv nachgeregelten Glucosekonzentration im Perfusat.

Die in der Zeichnung dargestellte Mikrodialyseanordnung besteht im wesentlichen aus einer in subkutanes Gewebe eines Probanden implantierbaren Mikrodialysesonde 10, einer Perfusionseinrichtung 12, 14 zur intervallweisen Perfusion der Mikrodialysesonde 10 mit glucosehaltigem Perfusat, einer Durchfluß-Meßzelle 16 zur Erfassung des Glucosegehalts im durchfließenden Dialysat, einer Regeleinrichtung 18, 20 zur Angleichung des Ausgangsgehalts der Glucose im Perfusat an den Glucosegehalt der Gewebeflüssigkeit und einer Auswerteeinheit 22 zur Bestimmung des Glucosegehalts der Gewebeflüssigkeit.

Die Mikrodialysesonde 10 weist eine Dialysemembran 24 auf, über welche ein Diffusionsaustausch von Glucose zwischen dem in der Sonde befindlichen Perfusat und der die Sonde umgebenden interstitiellen Flüssigkeit unter Gewinnung von Dialysat möglich ist. Hierzu ist in dem röhrenförmigen doppellumigen Sondengehäuse 25 ein zumindest teilweise von der Dialysemembran 24 begrenzter Durchflußkanal vorgesehen, welcher im proximalen Sondenbereich eintaßseitig mit einer Perfusatleitung 26 zur Einleitung von Perfusat und auslaßseitig mit einer Dialysatleitung 28 zur Ableitung des beim Dialysevorgang aus dem Perfusat gebildeten Dialysats verbunden ist. Das Dialysat läßt sich über die Dialysatleitung 28 zu der Meßzelle 16 und von dort in einen Auffangbehälter 30 weiterleiten. Geeignete Mikrodialysesonden der beschriebenen Art sind insbesondere aus der DE-A 33 42 170 bzw. US-PS 4,694,832 wohlbekannt und können von der in Solna, Schweden ansässigen Firma CMA/Microdialysis AB unter der Bezeichnung "CMA 60 Microdialysis Catheter" bzw. "CMA 70 Brain Microdialysis Catheter" erworben werden.

Zur intervallweisen Beaufschlagung der Mikrodialysesonde 10 mit glucosehaltigem Perfusat umfaßt die Perfusionseinrichtung einen Perfusatvorrat 12 und eine Fördereinheit 14. Der Perfusatvorrat 12 ist durch zwei gesonderte Reservoirs 32, 34 gebildet, von denen eines glucosefreie Perfusionsflüssigkeit 36 und das andere mit Glucose von vorgegebener Konzentration versetzte Perfusionsflüssigkeit 38 enthält. Die Glucosekonzentration der Flüssigkeit 38 beträgt zweckmäßig mehr als 4g/l, so daß durch Mischen der Flüssigkeiten 36, 38 in nachstehend beschriebener Weise der physiologische Bereich der Gewebeglucose im Perfusat abgedeckt werden kann. Um das Perfusat in dosierten Förderschüben von wenigen Mikrolitern durch die Mikrodialysesonde 10 und die nachgeordnete Meßzelle 16 zu fördern, ist eine intervallweise betriebene Schlauchpumpe 14 als Fördereinheit vorgesehen. Diese ist vorteilhafterweise in der Dialysatleitung 28 angeordnet, um während der Förderpausen die Mikrodialysesonde 10 gegenüber der extrakorporal angeordneten Meßzelle 16 abzusperren.

Die Meßzelle 16 weist einen von der durchströmenden Perfusionsflüssigkeit bzw. dem darin enthaltenen Dialysat beaufschlagten, elektrochemischenzymatisch arbeitenden Elektrodensensor 40 zur kontinuierlichen Signalerfassung auf. Der Sensor 40 weist mit dem als Elektrolyt dienenden Dialysat beaufschlagte nicht gezeigte Meßelektroden auf, über welche von dem Glucosegehalt des Dialysats linear abhängige Meßsignale als kontinuierlicher Meßstrom fortlaufend abgegriffen werden. Nähere Einzelheiten dieses Meßprinzips sind im Stand der Technik insbesondere aus der DE-A 44 01 400 bekannt, worauf hier ausdrücklich Bezug genommen wird. Es versteht sich, daß die Meßsignale auch den Glucosegehalt der Körperflüssigkeit wiederspiegeln, soweit in der Mikrodialysesonde ein vollständiger Ausgleich eines Konzentrationsgradienten zwischen dem Perfusat und der Körperflüssigkeit stattfindet bzw. der Angleichungsgrad bekannt ist.

Die Meßsignale des Sensors 40 werden in dem nachgeschalteten Meßumformer 42 elektronisch aufbereitet und über einen getakteten Analog-Digital-Wandler als zeitliche Abfolge von Digitalwerten in einen digital arbeitenden Regler 18 der Regeleinrichtung eingespeist. Der Regler 18 ist dabei durch einen Mikrocontroller realisiert, welcher zugleich die Auswerteeinheit 22 bildet. Ausgangsseitig ist der Regler 18 zur Einstellung des Ausgangsgehalts an Glucose im Perfusat mit einem Wegeventil 20 als Stellglied der Regeleinrichtung verbunden. Das Wegeventil 20 verbindet die Perfusatleitung 26 in einer federzentrierten ersten Schaltstellung mit dem glucosefreien Reservoir 32 und in der elektromagnetisch betätigten zweiten Schaltstellung mit dem glucosehaltigen Reservoir 34. Damit läßt sich in einem getakteten Betrieb durch geeignete Wahl der Schaltfrequenz das Mengenverhältnis der durch die Schlauchpumpe 14 angesaugten Flüssigkeiten 36, 38 und durch nachfolgend in der Perfusatleitung 26 stattfindendes Strömungsmischen die Glucosekonzentration im Perfusat als Regelgröße beeinflussen.

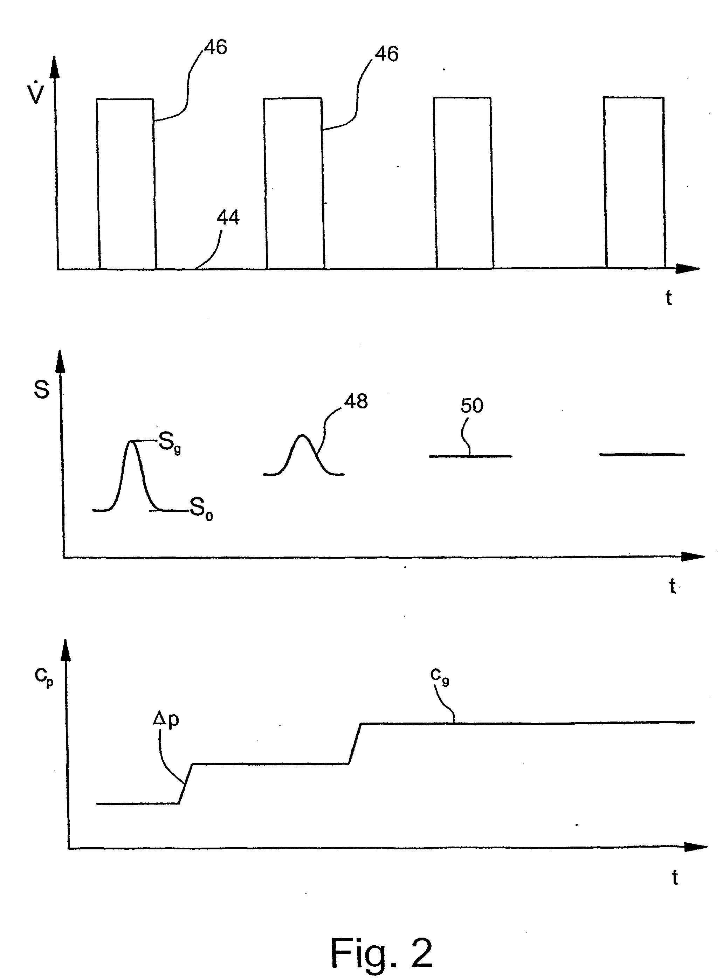

Beim Betrieb der Mikrodialyseanordnung wird gemäß dem oberen Diagramm in Fig. 2 das Perfusat in durch Ruhe- bzw. Dialyseintervalle 44 voneinander getrennten Transportintervallen 46 durch die Mikrodialysesonde 10 und die Meßzelle 16 gepumpt. Die Dialyseintervalle 44 können dabei so bemessen werden, daß der Glucosegehalt des in der Mikrodialysesonde 10 ruhenden Perfusatvolumens durch Diffusionsaustausch nahezu vollständig an die Gewebeglucose angeglichen wird. Während der Transportintervalle 46 hingegen bleibt die Glucosekonzentration im Perfusat aufgrund des raschen Durchtritts durch die Sonde im wesentlichen unverändert. Der Angleichungsgrad bzw. die "Recovery" hängt u.a. von der Verweildauer bzw. Fließgeschwindigkeit des Perfusats in der Mikrodialysesonde 10 ab. Bei dem in Fig. 2 gezeigten Ausführungsbeispiel beträgt die Dauer eines Dialyseintervalls 360 s bei unterbrochener Förderung, während die Dauer eines Transportintervalls 180 s bei einem Förderstrom $\dot{V}$ von 0,08 µl/s beträgt.

Bei jedem Förderschub wird das im vorangehenden Dialyseintervall gebildete Dialysat in einer Förder- bzw. Flüssigkeitssäule vollständig aus der Mikrodialysesonde 10 heraus zumindest in die Dialysatleitung 28 und vorzugsweise bis zur Meßzelle 16 verdrängt. Dementsprechend wird dort während der Transportintervalle 46 ein Signal S abgegriffen, das bei Konzentrationsunterschieden zwischen der Gewebe- und Perfusatglucose einen der Gewebeglucose entsprechenden Spitzen- bzw. Extremwert $S_g$ und einen der Ausgangskonzentration an Glucose im Perfusat entsprechenden Grundlinienwert $S_0$ zeigt (mittleres Diagramm in Fig. 2).

Zur Angleichung der Ausgangsglucose im Perfusat an die Gewebeglucose wird mittels der Auswerteeinheit 22 aus den Meßsignalen eine der Gewebeglucose entsprechende Führungsgröße abgeleitet und dem Regler 18 unter Bildung der Regeldifferenz gegenüber der Regelgröße, d.h. dem Momentanwert der Ausgangsglucose eingespeist. Dabei ist die Führungsgröße mit den Signalspitzen $S_g$ korreliert, während die Regelgröße durch den Grundlinienwert $S_0$ erfaßbar ist.

Für eine besonders einfache Regelung genügt es, wenn die Führungsgröße bzw. die Regeldifferenz durch eine qualitative Erkennung von Signalspitzen $S_g$ ermittelt und der Ausgangsgehalt der Glucose im Perfusat durch einen Zweipunktregelvorgang unstetig angepaßt wird. Dabei wird durch das Stellglied 20 der Glucose-Ausgangsgehalt $c_p$ bei Auftreten einer positiven Signalspitze (Signalpeak 48) um einen vorgegebenen Wert $\Delta p$ erhöht und bei einer negativen Spitze (Signaldip; nicht gezeigt) entsprechend reduziert. Für diesen Regelbetrieb reicht bereits eine geringe Angleichung bzw. Recovery (< 50%) während der Dialyseintervalle aus, so daß deren Dauer entsprechend verringert werden kann.

Das Stellsignal läßt sich bei gleichbleibenden Transportintervallen erst nach Ablauf eines Dialyseintervalls umsetzen. Um diese Totzeit zu vermeiden, ist es auch denkbar, bei einer Regelabweichung die Dauer des momentanen Transportintervalls zu verlängern, um sicherzustellen, daß die Perfusionsflüssigkeit mit dem nachgeregelten Glucosegehalt sogleich in die Mikrodialysesonde 10 gelangt.

Bei verschwindender Regelabweichung wird schließlich ein Konstantsignal 50 beobachtet, welches anzeigt, daß der Glucose-Ausgangsgehalt $c_p$ mit dem aktuellen Wert $c_g$ der Gewebeglucose übereinstimmt (Fig. 3 unten). Auf diese Weise ist es möglich, auf eine fehleranfällige unmittelbare Auswertung der Meßsignale zu verzichten und die Gewebeglucose mittelbar aus dem Gleichgewichtswert $c_p$ beim Auftreten eines Konstantsignals 50 zu bestimmen. Dies läßt sich ohne zusätzlichen Meßaufwand dadurch bewerkstelligen, daß der Ausgangsgehalt der Glucose im Perfusat mittels der Auswerteeinheit 22 aus der aktuellen Stellgröße, also der Schaltfrequenz des Ventils 20 gegebenenfalls durch Vergleich mit zugeordneten Kalibrierwerten bestimmt wird.

Eine Möglichkeit zur quantitativen Auswertung der Meßsignale besteht darin, daß die Führungsgröße durch Mustererkennung, d.h. durch Vergleich des aktuellen Signalverlaufs der Meßsignale mit in einem Speichermittel hinterlegten kalibrierten Signalmustern ermittelt wird. Alternativ kann die Regeldifferenz als Differenz des Spitzenwerts und des Grundlinienwerts des Signalverlaufs der Meßsignale bestimmt werden. In diesem Fall wird also der Ist-Wert der Regelgröße als Grundlinienwert $S_0$ durch Messung erfaßt.

Wie vorstehend ausgeführt, wird das Sensorsignal nur während der Transportintervalle ausgewertet. Der Signalverlauf umfaßt dabei einen mit der Dialysephase korrelierten Teil $S_g$, welcher proportional zum Gewebeglucosegehalt ist, und einen Teil $S_0$, welcher auf einen hohen Sondendurchfluß zurückführbar ist und nahezu (bis auf verbleibende Recovery-Effekte) die Ausgangskonzentration der Glucose im Perfusat wiederspiegelt. Um eine von Sensitivitätsschwankungen unabhängige Regelung zu ermöglichen, kann die Führungsgröße entsprechend dem Glucosegehalt c der Gewebeflüssigkeit gemäß der Beziehung

$$c = \frac{S_g}{S_0} \cdot c_0 \qquad (1)$$

bestimmt werden, wobei $c_0$ den - durch die Stellgröße bestimmbaren - momentanen Ausgangsgehalt der Glucose im Perfusat bezeichnet.

Weitere Einflußgrößen lassen sich dadurch berücksichtigen, daß die Gewebeglukosekonzentration bzw. die Führungsgröße nach der Beziehung

$$c = \left[\frac{S_g}{S_g \cdot (1-b) + b \cdot S_0} - 1\right] \cdot a \cdot c_0 + c_0 \qquad (2)$$

ermittelt wird, wobei a, b empirisch bestimmte Korrekturfaktoren zur Kompensation von Diffusions- und Mischungseffekten sowie verbleibenden Glucose-Angleichungseffekten zwischen Körper- und Perfusionsflüssigkeit während des Transportintervalls bezeichnen. Der Spitzenwert $S_g$ ist der Sensorwert zu einem definierten Zeitpunkt während des Transportintervalls. Dieser Zeitpunkt kann *in vitro* bestimmt werden und ist durch die Förderdauer des Dialysats von der Mikrodialysesonde 10 zu der Meßzelle 16 gegeben. $S_0$ kann als Mittelwert der Meßsignale in einem geeigneten Zeitabstand vor und nach dem Zeitpunkt von $S_g$ bestimmt werden. Für a, b = 1 geht die Beziehung (2) in (1) über.

Denkbar ist es, das vorstehend beschriebene Prinzip der selbstanpassenden Regelung des Glucosegehalts auch bei Messungen mit kontinuierlichem Perfusatstrom anzuwenden. Grundsätzlich ist diese Mikrodialysetechnik auch nicht auf subkutane Messungen am menschlichen Körper beschränkt.

Vielmehr können andere Körperflüssigkeiten wie Blut gegebenenfalls auch ex vivo untersucht werden.

**Patentansprüche**

1. Anordnung zur Konzentrationsbestimmung von Glucose in einer Körperflüssigkeit, insbesondere Gewebeflüssigkeit, mit einer Mikrodialysesonde (10) zum Diffusionsaustausch von Glucose mit umgebender Körperflüssigkeit, einer Perfusionseinrichtung (12,14) zur Perfusion der Mikrodialysesonde (10) mit glucosehaltigem Perfusat unter Gewinnung von Dialysat und einer der Mikrodialysesonde (10) nachgeordneten Meßzelle (16) zur Erfassung von mit dem Glucosegehalt des Dialysats korrelierten Meßsignalen, **gekennzeichnet durch** eine Regeleinrichtung (18,20) zur Angleichung des Ausgangsgehalts der Glucose im Perfusat an den Glucosegehalt der Körperflüssigkeit nach Maßgabe einer aus den Meßsignalen der Meßzelle (16) abgeleiteten Führungsgröße.

2. Anordnung nach Anspruch 1, **gekennzeichnet durch** eine Auswerteeinheit (22) zur Bestimmung des momentanen Ausgangsgehalts der Glucose im Perfusat bei verschwindender Regelabweichung als Maß für den Glucosegehalt der Körperflüssigkeit.

3. Anordnung nach Anspruch 1 oder 2, **dadurch gekennzeichnet, daß** der Ausgangsgehalt der Glucose im Perfusat aus der Stellgröße eines Stellglieds (20) der Regeleinrichtung (18,20) ermittelt wird.

4. Anordnung nach einem der Ansprüche 1 bis 3, **dadurch gekennzeichnet, daß** der Glucosegehalt des Perfusats vor der Einleitung in die Mikrodialysesonde (10) gemessen wird.

5. Anordnung nach einem der Ansprüche 1 bis 4, **dadurch gekennzeichnet, daß** die Perfusionseinrichtung einen Perfusatvorrat (12) sowie eine Fördereinheit (14) zur vorzugsweise intervallweisen Förderung von Perfusat aufweist.

6. Anordnung nach Anspruch 5, **dadurch gekennzeichnet, daß** der Perfusatvorrat (12) mindestens zwei gesonderte Reservoirs (32,34) zur Aufnahme von Pertusionsfiüssigkeiten (36,38) mit voneinander verschiedener Glucosekonzentration aufweist.

7. Anordnung nach Anspruch 5 oder 6, **dadurch gekennzeichnet, daß** der Perfusatvorrat (12) ein glucosefreie Perfusionsflüssigkeit (36) enthaltendes erstes Reservoir (32) und ein glucosehaltige Perfusionsflüssigkeit (38) enthaltendes zweites Reservoir (34) aufweist.

8. Anordnung nach einem der Ansprüche 1 bis 7, **dadurch gekennzeichnet, daß** die Regeleinrichtung einen vorzugsweise durch ein Mischventil oder getaktet schaltbares Wegeventil gebildeten Strömungsmischer (20) als Stellglied zur Einstellung des Ausgangsgehalts der Glucose im Perfusat aufweist.

9. Anordnung nach Anspruch 8, **dadurch gekennzeichnet, daß** der Strömungsmischer (20) zuströmseitig mit mindestens zwei Reservoirs (32,34) zur Zuleitung von Perfusionsflüssigkeiten mit voneinander verschiedenem Glucosegehalt verbunden ist und abströmseitig in eine zu der Mikrodialysesonde (10) führende Perfusatteitung (26) mündet.

10. Anordnung nach einem der Ansprüche 1 bis 9, **dadurch gekennzeichnet, daß** die Regeleinrichtung einen digital arbeitenden, vorzugsweise durch einen Mikrocontroller gebildeten Regler (18) aufweist.

11. Anordnung nach einem der Ansprüche 1 bis 10, **dadurch gekennzeichnet, daß** das Perfusat in alternierend aufeinanderfolgenden Transport- und Dialyseintervallen mit unterschiedlicher Fließgeschwindigkeit durch die Mikrodialysesonde (10) hindurchgeleitet wird, wobei die Fließgeschwindigkeit während der Transportintervalle höher ist als während der Dialyseintervalle.

12. Anordnung nach Anspruch 11, **dadurch gekennzeichnet, daß** die Fließgeschwindigkeit während der Transportintervalle so weit erhöht wird, daß der Ausgangsgehalt der Glucose im Perfusat beim Durchgang durch die Mikrodialysesonde (10) im wesentlichen erhalten bleibt, und daß während der Dialyseintervalle die Förderung unterbrochen oder zumindest die Fließgeschwindigkeit so verringert wird, daß die Glucosekonzentration im Dialysat an den Glucosegehalt der Körperflüssigkeit angenähert wird.

13. Anordnung nach einem der Ansprüche 1 bis 12, **dadurch gekennzeichnet, daß** die Führungsgröße durch Integration oder Differentiation des zeitlichen Verlaufs der Meßsignale ermittelt wird.

14. Anordnung nach einem der Ansprüche 11 bis 13, **dadurch gekennzeichnet, daß** die Führungsgröße durch eine qualitative Erkennung von Signalspitzen im zeitlichen Verlauf der Meßsignale ermittelt wird.

15. Anordnung nach einem der Ansprüche 11 bis 14, **dadurch gekennzeichnet, daß** die Führungsgröße durch Vergleich des aktuellen Signalverlaufs der Meßsignale mit in einem Speichermittel hinterlegten kalibrierten Signalmustem ermittelt wird.

16. Anordnung nach einem der Ansprüche 11 bis 15, **dadurch gekennzeichnet, daß** die Führungsgröße aus dem Spitzenwert des Signalverlaufs der Meßsignale während eines jeden Transportintervalls ermittelt wird.

17. Anordnung nach einem der Ansprüche 11 bis 16, **dadurch gekennzeichnet, daß** die Führungsgröße entsprechend dem Glucosegehalt c der Körperflüssigkeit gemäß der Beziehung

$$c = \left[ \frac{S_g}{S_g \cdot (1\text{-}b) + b \cdot S_0} - 1 \right] \cdot a \cdot c_0 + c_0$$

bestimmt wird, wobei $S_g$ den Spitzenwert und $S_0$ den Grundlinienwert der Meßsignale während eines Transportintervalls, $c_0$ den momentanen Ausgangsgehalt der Glucose im Perfusat und a, b empirisch bestimmte Korrekturfaktoren zur Kompensation von Diffusions- und Mischungseffekten sowie verbleibenden Recovery-Effekten während eines Transportintervalls bezeichnen.

18. Anordnung nach einem der Ansprüche 1 bis 17, **dadurch gekennzeichnet, daß** der Ausgangsgehalt der Glucose im Perfusat durch einen Zweipunktregelvorgang unstetig geregelt wird, wobei bei einer Regelabweichung der Ausgangsgehalt der Glucose im Perfusat um einen vorgegebenen Stellwert verändert wird.

19. Anordnung zur Konzentrationsbestimmung von Glucose in einer Körperflüssigkeit, insbesondere Gewebeflüssigkeit, mit einer in die Körperflüssigkeit eingreifenden Mikrodialysesonde (10), mindestens zwei Reservoirs (32,34) zur Aufnahme von Perfusionsflüssigkeiten (36,38) mit voneinander verschiedenem Glucosegehalt, einer Fördereinheit (14) zur Perfusion der Mikrodialysesonde (10) mit glucosehaltigem Perfusat unter Gewinnung von Dialysat und einer der Mikrodialysesonde. (10) nachgeordneten Durchfluß-Meßzelle (16) zur Erfassung von mit dem Glucosegehalt des Dialysats korrelierten Meßsignalen, **gekennzeichnet durch** eine eingangsseitig mit der Meßzelle (16) verbundene Regeleinrichtung (18,20), die eine zuströmseitig mit den Reservoirs (32,34) und abströmseitig mit der Mikrodialysesonde (10) verbundenen Strömungsmischer (20) als Stellglied zur Regelung des Ausgangsgehalts der Glucose im Perfusat aufweist.

## Claims

1. Apparatus for determining the glucose concentration in a body fluid, in particular tissue fluid, comprising a microdialysis probe (10) for the diffusion exchange of glucose with surrounding body fluid, a perfusion device (12, 14) for perfusing the microdialysis probe (10) with glucose-containing perfusate to obtain dialysate and a measuring cell (16) located after the microdialysis probe (10) for detecting measurement signals that correlate with the glucose content of the dialysate, **characterized by** a control device (18, 20) which adjusts the starting content of glucose in the perfusate to the glucose content of the body fluid in accordance with a command variable derived from the measurement signals of the measuring cell (16).

2. Apparatus as claimed in claim 1, **characterized by** an evaluation unit (22) to determine the momentary starting content of glucose in the perfusate when the control deviation is negligible as a measure for the glucose content of the body fluid.

3. Apparatus as claimed in claims 1 or 2, **characterized in that** the starting content of glucose in the perfusate is determined from the adjusting variable of an adjuster (20) of the control device (18, 20).

4. Apparatus as claimed in one of the claims 1 to 3, **characterized in that** the glucose content of the perfusate is measured before it is passed into the microdialysis probe (10).

5. Apparatus as claimed in one of the claims 1 to 4, **characterized in that** the perfusion device has a perfusate store (12) and a transport unit (14) for the preferably intermittent transport of perfusate.

6. Apparatus as claimed in claim 5, **characterized in that** the perfusate store (12) has at least two separate reservoirs (32, 34) to hold perfusion liquids (36, 38) with different glucose concentrations.

7. Apparatus as claimed in claim 5 or 6, **characterized in that** the perfusate store (12) has a first reservoir (32) containing a glucose-free perfusion liquid (36) and a second reservoir (34) containing a glucose-containing perfusion liquid (38).

8. Apparatus as claimed in one of the claims 1 to 7, **characterized in that** the control device has a flow mixer (20) preferably comprising a mixing valve or clock-pulsed directional control valve as an adjuster to adjust the starting content of glucose in the perfusate.

9. Apparatus as claimed in claim 8, **characterized in that** the inlet side of the flow mixer (20) is connected to at least two reservoirs (32, 34) for feeding in perfusion fluids with different glucose contents and the outlet side of the flow mixer (20) is connected to a perfusate tube (26) leading to the microdialysis probe (10).

10. Apparatus as claimed in one of the claims 1 to 9, **characterized in that** the control device has a digitally operated controller (18) preferably in the form of a microcontroller.

11. Apparatus as claimed in one of the claims 1 to 10, **characterized in that** the perfusate is passed through the microdialysis probe (10) in alternating successive transport and dialysis intervals at different flow rates, the flow rate during the transport intervals being higher than during the dialysis intervals.

12. Apparatus as claimed in claim 11, **characterized in that** the flow rate during the transport intervals is increased to such an extent that the starting content of glucose in the perfusate during passage through the microdialysis probe (10) remains essentially constant and that during the dialysis intervals the transport is interrupted or at least the flow rate is reduced to such an extent that the glucose concentration of the dialysate approximates the glucose content of the body fluid.

13. Apparatus as claimed in one of the claims 1 to 12, **characterized in that** the command variable is determined by integration or differentiation of the time course of the measurement signals.

14. Apparatus as claimed in one of the claims 11 to 13, **characterized in that** the command variable is determined by qualitative detection of signal peaks in the time course of the measurement signals.

15. Apparatus as claimed in one of the claims 11 to 14, **characterized in that** the command variable is determined by comparing the actual signal curve of the measurement signals with calibrated signal patterns stored in a storage medium.

16. Apparatus as claimed in one of the claims 11 to 15, **characterized in that** the command variable is determined from the peak value of the signal time course of the measurement signals during each transport interval.

17. Apparatus as claimed in one of the claims 11 to 16, **characterized in that** the command variable can be determined according to the glucose content c of the body fluid according to the relationship

$$c = \left[ \frac{S_g}{S_g \cdot (1-b) + b \cdot S_0} - 1 \right] \cdot a \cdot c_0 + c_0$$

in which $S_g$ denotes the peak value and $S_0$ denotes the base line value of the signals measured during a transport interval and $c_0$ represents the momentary starting content of glucose in the perfusate and a, b represent empirically determined correction factors for compensation of diffusion and mixing effects as well as remaining recovery effects during a transport interval.

18. Apparatus as claimed in one of the claims 1 to 17, **characterized in that** the starting content of glucose in the perfusate is regulated discontinuously by a two-point control process in which the starting content of glucose in the perfusate is changed by a predetermined adjustment value when there is a control deviation.

19. Apparatus for determining the glucose concentration in a body fluid, in particular tissue fluid, comprising a microdialysis probe (10) inserted into the body fluid, at least two reservoirs (32, 34) for holding perfusion liquids (36, 38) with different glucose contents, a transport unit (14) to perfuse the microdialysis probe (10) with glucose-containing perfusate to obtain dialysate and a flow-through measuring cell (16) located downstream of the microdialysis probe (10) to register measurement signals that correlate with the glucose content of the dialysate, **characterized by** a control device (18, 20) connected on the input side to the measuring cell (16) which control device has a flow mixer (20) connected on the inlet side to the reservoirs (32, 34) and on the outlet side to the microdialysis probe (10) which acts as an adjuster to regulate the starting content of glucose in the perfusate.

## Revendications

1. Dispositif pour déterminer la concentration de glucose dans un fluide anatomique, en particulier un fluide tissulaire, avec une sonde de microdialyse (10) permettant l'échange par diffusion de glucose avec le fluide anatomique environnant, un appareil de perfusion (12, 14) destiné à perfuser la sonde de microdialyse (10) avec un produit de la perfusion dénommé perfusé contenant du glucose en recueillant le produit de la dialyse dénommé dialysé, et une cellule de mesure (16) disposée après la sonde de micro-dialyse (10) et destinée à enregistrer des signaux de mesure corrélés à la teneur en glucose du produit de la dialyse, **caractérisé par** un dispositif de régulation (18, 20) permettant d'adapter la teneur de départ en glucose dans le perfusé à la teneur en glucose du fluide anatomique au prorata d'une grandeur de référence dérivée des signaux de mesure de la cellule de mesure (16).

2. Dispositif selon la revendication 1, **caractérisé par** une unité d'exploitation (22) permettant de déterminer la teneur de départ instantanée en glucose dans le perfusé lorsque la déviation de réglage disparaît comme mesure de la teneur en glucose du fluide anatomique.

3. Dispositif selon la revendication 1 ou 2, **caractérisé en ce que** la teneur de départ en glucose dans le perfusé est déterminée à partir de la grandeur de réglage d'un organe de commande (20) du dispositif de régulation (18, 20).

4. Dispositif selon l'une des revendications 1 à 3, **caractérisé en ce que** la teneur en glucose du perfusé est mesurée avant introduction dans la sonde de micro-dialyse (10).

5. Dispositif selon l'une des revendications 1 à 4, **caractérisé en ce que** l'installation de perfusion possède un réservoir de perfusé (12) et une unité de transport (14) permettant le transport, de préférence à intervalles, du perfusé.

6. Dispositif selon la revendication 5, **caractérisé en ce que** le réservoir de perfusé (12) présente au moins deux réservoirs séparés (32, 34) permettant de recevoir des liquides de perfusion (36, 38) présentant des concentrations de glucose qui diffèrent l'une de l'autre.

7. Dispositif selon la revendication 5 ou 6, **caractérisé en ce que** le réservoir de perfusé (12) possède un premier réservoir (32) contenant un liquide de perfusion sans glucose (36) et un second réservoir (34) contenant un liquide de perfusion qui contient du glucose.

8. Dispositif selon l'une des revendications 1 à 7, **caractérisé en ce que** l'appareil de régulation présente un mélangeur de flux (20) formé de préférence par une vanne de mélange ou une vanne à canaux commutable synchronisée en tant qu'organe de commande (20) permettant de régler la teneur de départ en glucose dans le perfusé.

**9.** Dispositif selon la revendication 8, **caractérisé en ce que** le mélangeur (20) est relié côté amont à au moins deux réservoirs (32, 34) d'amenée des liquides de perfusion présentant des teneurs en glucose qui diffèrent, et débouche en aval dans une conduite de perfusé (26) menant à la sonde de microdialyse (10).

**10.** Dispositif selon l'une des revendications 1 à 9, **caractérisé en ce que** l'appareil de régulation présente un régulateur numérique (18) de préférence formé par un microcontrôleur.

**11.** Dispositif selon l'une des revendications 1 à 10, **caractérisé en ce que** le perfusé, selon des intervalles de transport et de dialyse qui se succèdent par alternance, est conduit à travers la sonde de microdialyse (10) selon une vitesse d'écoulement différente, la vitesse d'écoulement pendant les intervalles de transport étant supérieure à celle pendant les intervalles de dialyse.

**12.** Dispositif selon la revendication 11, **caractérisé en ce que** la vitesse d'écoulement pendant les intervalles de transport est augmentée de façon telle que la teneur de départ en glucose dans le perfusé lors du passage à travers la sonde de microdialyse (10) est essentiellement maintenue, et **en ce que**, pendant les intervalles de dialyse, le déplacement est interrompu ou au moins la vitesse d'écoulement réduite de façon telle que la concentration en glucose dans le produit dialysé s'approche de la teneur en glucose du fluide anatomique.

**13.** Dispositif selon l'une des revendications 1 à 12, **caractérisé en ce que** la grandeur de référence est déterminée par intégration ou différenciation de l'évolution temporelle des signaux de mesure.

**14.** Dispositif selon l'une des revendications 11 à 13, **caractérisé en ce que** la grandeur de référence est déterminée par une reconnaissance qualitative de pics de signaux lors de l'évolution temporelle des signaux de mesure.

**15.** Dispositif selon l'une des revendications 11 à 14, **caractérisé en ce que** la grandeur de référence est déterminée par comparaison de l'évolution réelle des signaux de mesure avec des modèles de signaux étalonnés consignés dans une mémoire.

**16.** Dispositif selon l'une des revendications 11 à 15, **caractérisé en ce que** la grandeur de référence est déterminée à partir de la valeur de pic de l'évolution des signaux de mesure pendant chaque intervalle de transport.

**17.** Dispositif selon l'une des revendications 11 à 16, **caractérisé en ce que** la grandeur de référence est déterminée en fonction de la teneur en glucose c du fluide anatomique selon l'équation

$$c = \left[\frac{S_g}{S_g \cdot (1-b) + b \cdot S_0} - 1\right] \cdot a \cdot c_0 + c_0$$

où $S_g$ est la valeur de pic et $S_0$ la valeur de ligne de base des signaux de mesure pendant un intervalle de transport, $c_0$ la teneur instantanée de départ en glucose dans le perfusé et a, b des facteurs de correction déterminés empiriquement pour compenser les effets de diffusion et de mélange ainsi que les effets de récupération restant pendant un intervalle de transport.

**18.** Dispositif selon l'une des revendications 1 à 17, **caractérisé en ce que** la teneur de départ en glucose dans le perfusé est régulée de façon non continue par un procédé de régulation en deux points, où en cas de déviation de réglage, la teneur de départ en glucose dans le perfusé est modifiée d'une valeur de consigne prédéfinie.

**19.** Dispositif de détermination de la concentration de glucose dans un fluide anatomique, en particulier un fluide tissulaire, comportant une sonde de microdialyse (10) s'insérant dans le fluide anatomique, au moins deux réservoirs (32, 34) destinés à recevoir des liquides de perfusion (36, 38) ayant des teneurs en glucose qui diffèrent, une unité de transport (14) permettant la perfusion de la sonde de microdialyse (10) avec le perfusé contenant du glucose en recueillant le produit dialysé, et une cellule de mesure de débit (16) placée après la sonde de microdialyse (10) permettant d'enregistrer des signaux de mesure corrélés à la teneur en glucose du produit dialysé, **caractérisé par** un dispositif de régulation (18, 20) relié côté entrée à la cellule de mesure (16), lequel dispositif présente, en tant qu'organe de commande permettant de réguler la teneur de départ en glucose dans le perfusé, un mélangeur (20) relié en amont aux réservoirs (32, 34) et en aval à la sonde de microdialyse (10).

Fig. 1

Fig. 2